# EUROPÄISCHE PATENTANMELDUNG

(11) **EP 3 225 236 A1**
(43) Veröffentlichungstag der Anmeldung: **04.10.2017**
(21) Anmeldenummer: 17164084.0
(22) Anmeldetag: 31.03.2017
(51) Int. Cl.: A61K 9/28, A61K 9/10, A61K 47/02, A61P 35/00, A61K 33/24, A61K 9/50

(54) **PHOTOKATALYTISCH AKTIVE PARTIKEL MIT EINER MODIFIZIERTEN OBERFLÄCHE UND VERFAHREN ZUR HERSTELLUNG VON DISPERSIONEN DIESER PARTIKEL**

(30) Priorität: 31.03.2016 DE 102016205389
(71) Anmelder: Fraunhofer-Gesellschaft zur Förderung der angewandten Forschung e.V., 80686 München (DE)
(72) Erfinder: DEMBSKI, Sofia, 97078 Würzburg (DE); KOCH, Susanne, 97318 Kitzingen (DE); PROBST, Jörn, 97273 Kürnach (DE); HERBIG, Bettina, 97250 Erlabrunn (DE)
(74) Vertreter: Pfenning, Meinig & Partner mbB

(57) **Zusammenfassung**

Die vorliegende Erfindung betrifft photokatalytisch aktivierte Partikel mit einer Partikeloberfläche, die mit mindestens einem Carbonsäurederivat mit mindestens einer Ethoxygruppe modifiziert ist und die als Arzneimittel eingesetzt werden können sowie Dispersionen dieser Partikel und Verfahren zur Herstellung der Partikeldispersionen.

## Beschreibung

Die vorliegende Erfindung betrifft photokatalytisch aktivierte Partikel mit einer Partikeloberfläche, die mit mindestens einem Carbonsäurederivat mit mindestens einer Ethoxygruppe modifiziert ist und die als Arzneimittel eingesetzt werden können sowie Dispersionen dieser Partikel und Verfahren zur Herstellung der Partikeldispersionen.

An der Oberfläche von herkömmlichen photokatalytisch aktivierten Partikeln befinden sich Elektronen-Loch-Paare, die an der Luft und in Gegenwart von Feuchtigkeit reagieren. Mit Sauerstoff als Elektronenakzeptor bilden sie Superoxid-Ionen O₂⁻ aus, während mit Hydroxidionen OH⁻ als Elektronendonor Hydroxyl-Radikale OH· entstehen. Beide Spezies sind hochreaktiv und können mit beliebigen organischen Molekülen reagieren und diese dabei zerstören.

Dieses Reaktionsverhalten wird in vorteilhafter Weise zum Abtöten von Tumorzellen und Bakterien (Mikroorganismen) genutzt. Dabei müssen jedoch üblicherweise Dispersionen von photokatalytisch aktiven Partikeln im nicht-aktivierten Zustand zu den Zellkulturen gegeben werden, welche die malignen Tumorzellen enthalten. Erst nach der Wechselwirkung zwischen Partikel und Zellen (z.B. nach der Resorption) werden die Partikel durch UV-Bestrahlung aktiviert. Durch den oben beschriebenen Reaktionsmechanismus werden dann die Zellen, welche die Partikel aufgenommen hatten, vollständig zerstört.

Der Nachteil bei diesem herkömmlichen Verfahren ist jedoch, dass nur auf oberflächliche Tumore oder einer Gewebestrukturen eingewirkt werden kann. Innere, nicht-invasive Anwendungen sind nicht möglich, da UV-Licht das menschliche Gewebe nicht durchdringt. Darüber hinaus ist es nachteilig, dass sich die UV-Strahlung selbst schädigend auf das Gewebe auswirkt.

Um eine Schädigung des Gewebes durch UV-Strahlung zu vermeiden, wurden die Partikel bisher so modifiziert, dass sie mittels längerwelligen Lichts aktiviert werden können.

In Feng et al. (G. Feng, S. Liu, Z. Xiu, et al. Visible Light Photocatalytic Activities of TiO2 Nanocrystals Doped with Upconversion Luminescence Agent, Journal of Physical Chemistry C, 2008, Vol. 112, pp. 13692-13699) und in Wang, Shang, Zheng et al. (W. Wang, Q. Shang, W. Zheng, et al. A Novel Near-Infrared Antibacterial Material Depending on the Upconverting Property of Er3+-Yb3+-Fe3+ Tridoped TiO2 Nanopowder, Journal of Physical Chemistry C, 2010, Vol. 114, pp. 13663-13669) wurde dies beispielsweise durch eine Dotierung der Partikel erreicht. Die Dotierung resultiert in einer Vergrößerung der Halbleiter-Bandlücke und verschiebt die Anregungswellenlänge für die Photokatalyse in den längerwelligen Bereich des Lichtspektrums (z.B. in den sichtbaren oder in den Infrarot-Bereich). Eine Bestrahlung mit schädlichem UV-Licht ist somit nicht mehr notwendig.

Eine andere Herangehensweise ist die Konstruktion von Kern-Schale-Partikeln, die bei Wang, Huang, Ni et al. (W. Wang, W. Huang, Y. Ni, et al. Different Upconversion Properties of ß-NaYF:Yb3+,Tm3+/Er3+ in Affecting the Near-Infrared-Driven Photocatalytic Activity of High-Reactive TiO2, ACS Applied Materials & Interfaces. 2014, Vol. 6, pp. 340-348) vorgeschlagen wurde.

Diese Ansätze vermeiden zwar die Schädigung des Gewebes durch UV-Strahlung, innere Anwendungen werden dadurch jedoch auch nicht ermöglicht, da sichtbares und infrarotes Licht keine hohe Eindringtiefe in die Gewebestruktur erreicht.

Ausgehend von dem Stand der Technik war es demnach Aufgabe der vorliegenden Erfindung, photokatalytisch aktivierte Partikel bereitzustellen, die es erlauben, in nicht-invasiver Weise Tumorzellen abzutöten, die im Inneren einer Gewebestruktur oder eines Zellhaufens liegen. Die bereitzustellenden Partikel sollten es außerdem ermöglichen, Tumorzellen ohne eine Bestrahlung des Gewebes mit Licht, insbesondere UV-Licht, abzutöten.

Diese Aufgabe wird durch die photokatalytisch aktivierten Partikel mit den Merkmalen des Patentanspruches 1 gelöst, die in einer Dispersion mit den Merkmalen des Patentanspruches 8 enthalten sind. Patentanspruch 9 zeigt zusätzlich ein Verfahren zur Herstellung dieser Dispersion auf und Anspruch 14 betrifft über das erfindungsgemäße Verfahren hergestellte Partikel.

Die erfindungsgemäßen photokatalytisch aktivierten Partikel weisen einen durchschnittlichen Durchmesser zwischen 5 nm und 100 nm und eine Partikeloberfläche auf, die mit mindestens einem Carbonsäurederivat mit mindestens einer Ethoxygruppe modifiziert ist, wobei die Partikel mittels UV-Licht aktiviert wurden und durch die hierbei freigesetzten Radikale tumortoxische Eigenschaften aufweisen.

Im nicht-aktivierten Zustand sind die photokatalytisch aktiven Partikel bis zu einer Konzentration von 500 mg/ml nicht-toxisch. Durch UV-Licht Bestrahlung können die Partikel in einen anhaltenden aktivierten, tumortoxischen Zustand angeregt werden. Dieser ermöglicht, dass die Partikel vor der Zugabe zu den Zellen und der Resorption durch die Zellen ex-vivo, d.h. außerhalb eines lebenden Organismus und im Sinne vorliegender Erfindung insbesondere auch in Abwesenheit von Zellen, Gewebe oder Organen, mittels UV-Licht Bestrahlung aktiviert werden und erst im Anschluss daran zu den Zellen gegeben werden. Sie können dann zu den Tumorzellen im Inneren eines Zellhaufens oder einer Gewebestruktur gelangen und diese abtöten. Eine UV-Licht-Bestrahlung der Zellen entfällt.

Der durchschnittlichen Durchmesser der Partikel ist mit 5 bis 100 nm so gewählt, dass die Partikel gewebegängig sind und von den Zellen resorbiert werden können.

Des Weiteren ist bevorzugt, dass die Partikel ein Halbleitermaterial, besonders bevorzugt ein Halbleitermaterial ausgewählt aus der Gruppe bestehend aus Titanoxid, insbesondere Titanoxid in der Anatas- oder Rutil-Modifikation, Zinkoxid, Zinnoxid, Zirkonoxid, Cadmiumsulfid, Niobiumoxid und Mischungen hiervon enthalten.

Die Auswahl des Materials hat dabei einen direkten Einfluss auf die Wellenlänge des Anregungslichtes. Bei Titandioxid in der Rutil-Modifikation ist die Bandlücke bei 3,0 eV, was Licht mit einer Wellenlänge von ca. 410 nm entspricht. Wird Rutil in der Anatas-Modifikation verwendet, ist die Anregungswellenlänge kürzer und liegt bei ca. 380 nm, da die Bandlückenenergie hier 3,2 eV beträgt.

In einer Ausführungsform der Erfindung ist bevorzugt, dass das mindestens eine Carbonsäurederivat des Partikels maximal 15 Ethoxygruppen, besonders bevorzugt maximal 4 Ethoxygruppen, enthält. Nur wenn die Oberfläche des Partikels diese Maßgabe erfüllt, ist eine optimal tumortoxische Wirkung zu erwarten. Bei weniger oder mehr als der spezifizierten Anzahl an Ethoxygruppen wird im Vergleich dazu wieder eine Abnahme der tumortoxische Wirkung beobachtet.

Vorteilhafterweise weisen die erfindungsgemäßen Partikel nach einer mindestens fünfstündigen Aktivierung mittels UV-Licht-Bestrahlung gegenüber malignen humanen Zellen, bevorzugt Hypopharynxkarzinomzellen, besonders bevorzugt FaDu-Zellen, eine mittlere inhibitorische Konzentration IC₅₀ von maximal 150 µg/ml auf. Es ist besonders bevorzugt, wenn die Partikel nach derselben Aktivierungszeit mittels UV-Licht-Bestrahlung gegenüber nicht-malignen humanen Zellen, bevorzugt humanen mesenchymalen Knochenmarksstammzellen, eine größere mittlere inhibitorische Konzentration IC₅₀ aufweisen als gegenüber malignen Zellen, insbesondere eine IC₅₀ von mindestens 200 µg/ml.

In einer bevorzugten Ausführungsvariante werden bei einer Konzentration von 200 µg/ml 70% maligner Tumorzellen abgetötet. Besonders bevorzugt ist es, wenn gleichzeitig bei einer Konzentration von 200 µg/ml nur 30% der nicht-malignen humanen Zellen absterben.

In dieser Weise kann sichergestellt werden, dass gesunde Zellen bei der Zugabe der aktiven Partikel zu einer Mischung aus gesunden und malignen Zellen nicht im gleichen Ausmaß zerstört werden wie die malignen Tumorzellen.

In einer bevorzugten Ausführungsvariante der Erfindung werden die Partikel als Arzneimittel, insbesondere als Tumor-Therapeutikum oder als antimikrobiell wirksame Substanz eingesetzt.

Gemäß einer anderen bevorzugten Ausführungsform vorliegender Erfindung erfolgt die Aktivierung der Partikel mittels UV-Licht ex-vivo, besonders bevorzugt erfolgt diese Aktivierung in einer wässrigen Dispersion der Partikel.

Nach einer weiteren bevorzugten Ausführungsform erfolgt die Aktivierung der erfindungsgemäßen Partikel mit UVA-Licht, bevorzugt mit Licht des Wellenlängenbereiches von 315 bis 380 nm und besonders bevorzugt mit Licht der Wellenlänge 366 nm. Dabei ist es weiterhin bevorzugt, dass die Intensität der UV-Strahlung im Bereich von 0,1 bis 2 mW/cm² und bevorzugt von 0,3 bis 0,5 mW/cm² liegt. Außerdem ist es bevorzugt wenn die Aktivierung über einen Zeitraum von 5 bis 50 h, bevorzugt von 12 bis 24 h erfolgt. Gemäß einer bevorzugten Ausführungsform vorliegender Erfindung erfolgt die Aktivierung durch eine kumulierte Anwendung der vorgehend beschriebenen Maßnahmen.

Die erfindungsgemäß ebenfalls bereitgestellte, wässrige Dispersion enthält die vorangehend beschriebenen photokatalytischen aktiven Partikel.

Das Verfahren zur Herstellung dieser wässrigen Dispersion von photokatalytische aktiven Partikeln, umfasst die folgenden Verfahrensschritte:
a) Herstellen eines Sols aus einer metallorganischen Verbindung, einer Säure und Wasser,
b) Umsetzen des Sols bei Temperaturen von 160 bis 200°C zu einem partikelhaltigen Gel,
c) Verdünnen des Gels zu einer Dispersion mit einer Partikelkonzentration zwischen 0 und 20 Gew.-% und
d) Bestrahlen der Dispersion mit UV-Licht.

Dabei ist es bevorzugt, wenn die metallorganische Verbindung in Schritt a) aus der Gruppe bestehend aus
- Titanhalogeniden ausgewählt aus der Gruppe bestehend aus TiCl₄, TiOCl₂ und Mischungen hiervon
- Titanalkoxide, bevorzugt Titanalkoxide mit der allgemeinen Formel Ti(OR)₄, wobei R ein geradkettiger, verzweigter oder cyclischer Alkylrest mit 1 bis 6 Kohlenstoffatomen ist, besonders bevorzugt Titanalkoxide ausgewählt aus der Gruppe bestehend aus Titanium(IV)butoxid, Titanium(IV)ethoxid, Titanium(IV)propoxid, Titanium(IV)isopropoxid, Titanium(IV)tert-butoxid, Titanium(IV)methoxid sowie
- Mischungen hiervon
ausgewählt ist.

In einer bevorzugten Verfahrensvariante ist die Säure in Schritt a) ein Carbonsäurederivat, insbesondere ausgewählt aus der Gruppe bestehend aus Methoxypolyethylenglycol-Essigsäure, Methoxypolyethylenglycol-Propionsäure, O-Methyl-O'-succinylpolyethylenglycol, O-[2-(3-Succinyl-amino)ethyl]-O'-methyl-polyethylenglycol, O-(2-Carboxyethyl)-O'-methyl-undecaethylenglycol, 2-[2-(2-methoxyethoxy)ethoxy]-essigsäure, p-Toluolsulfonsäure und Mischungen hiervon, wobei die Säure in Schritt a) bevorzugt in einer Konzentration von 1 bis 100 mg/mL, besonders bevorzugt von 6 bis 25 mg/mL, vorliegt.

Das Verfahren zur Herstellung der Dispersion kann darüber hinaus dadurch gekennzeichnet sein, dass dem Sol in Schritt a) weitere Partikel, bevorzugt Silica-Kern-Schale-Partikel oder Eisenoxid-Kern-Schale-Partikel, zugegeben werden.

Das in Schritt b) hergestellte Gel wird in Schritt c) bevorzugt auf eine Partikelkonzentration von 0,1 bis 10 mg/mL, besonders bevorzugt auf eine Konzentration von 1 bis 5 mg/mL, verdünnt.

Der Dispersion in Schritt c) wird vorteilhafterweise ein Polycarboxylether, bevorzugt in einer Menge von 0,4 mg/Partikel, zugegeben.

Außerdem kann das UV-Licht, mit dem die Dispersion in Verfahrensschritt d) bestrahlt wird, aus UVA-Licht, bevorzugt aus Licht des Wellenlängenbereiches von 315 bis 380 nm, besonders bevorzugt aus Licht der Wellenlänge 366 nm, bestehen.

Die Wellenlänge richtet sich dabei danach, auf welchem Metall die eingesetzte metallorganischen Verbindung basiert.

Das UV-Licht, mit dem die Dispersion bestrahlt wird, weist bevorzugt eine Intensität von 0,1 bis 2 mW/cm², besonders bevorzugt von 0,3 bis 0,5 mW/cm², auf.

In einer vorteilhaften Variante des Herstellverfahrens wird die Dispersion in Schritt d) über einen Zeitraum von 5 bis 50 h, bevorzugt von 12 bis 24 h, bestrahlt.

Anhand der nachfolgenden Beispiele soll das erfindungsgemäße Verfahren näher erläutert werden, ohne dieses auf diese Ausführungsformen einschränken zu wollen.

### Beispiel 1

Für die Synthese der Partikel werden Carbonsäurederivate wie z.B. Methoxypolyethylenglycol-Essigsäure, Methoxypolyethylenglycol-Propionsäure, O-Methyl-O'-succinylpolyethylenglycol, O-[2-(3-Succinyl-amino)ethyl]-O'-methyl-polyethylenglycol, O-(2-Carboxyethyl)-O'-methyl-undecaethylenglycol, 2-[2-(2-methoxyethoxy)ethoxy]-essigsäure, p-Toluolsulfonsäure und Mischungen hiervon sowie Titanium(IV)butoxid als Edukte gewählt. Sie werden gemischt und mit Wasser hydrolysiert. Dabei sind die Molverhältnisse der Edukte 1,00/1,89/3,33 (Titanium(IV)butoxid/ Carbonsäurederivat/Wasser).

Nach der Entfernung flüchtiger Bestandteile durch Vakuumrotationsevaporation wird die hochviskose Flüssigkeit mit Wasser verdünnt, um ein Sol von 12 Gewichtsprozent zu erhalten. Dieses wird im Autoklaven bei 160-200 °C für 1-16 h behandelt. Das dadurch erhaltene Gel wird in Wasser redispergiert und dadurch auf 3 Gewichtsprozent verdünnt. Anschließend wird die saure Partikeldispersion mit einer 0,8 µm Membran druckfiltriert. Die Dispersion wird nun auf 3 mg/ml mit Wasser verdünnt und mittels Ultraschall dispergiert. 2 ml der wässrigen TiO₂-Partikel mit einer Konzentration von 3 mg/ml werden 24 h mit einer UV-Lampe (366 nm und 0,3-0,5 mW/cm²) bestrahlt. Dann wird die Partikeldispersion mit 1 M NaOH auf den pH-Wert 7 eingestellt und mit RPMI-Medium auf eine Konzentration von 0,3 mg/ml aufgefüllt. Nach Redispersion der Partikel durch Schütteln des Behältnisses, wird eine Konzentrationsreihe von 10 bis 400 µg/ml angesetzt und auf die Zellen gegeben. Als Zellen werden maligne Zellen wie die Hypopharynxkarzinomzelllinie FaDu und nicht-maligne Zellen wie humane mesenchymale Knochenmarksstammzellen gewählt. Die Zellen werden 24 h mit den Partikeln inkubiert. Nach dieser Zeit wird die Viabilität der behandelten Zellen mit Hilfe eines 3-(4,5-Dimethylthiazol-2-yl)-2,5-diphenyltetrazoliumbromid (MTI) Tests ermittelt. Die mittlere inhibitorische Konzentration (IC₅₀) der Partikel gegenüber den Tumorzellen beträgt 100-150 µg/ml, während die IC₅₀ der Partikel gegenüber nicht-malignen Zellen mindestens 200 µg/ml beträgt.

### Beispiel 2

Die Herstellung der Partikel wird wie in Beispiel 1 durchgeführt bis nach der Ultraschalldispergierung und Filtration der Proben. Anschließend werden die Partikel mit einem kommerziellen Polycarboxylatether, bevorzugt Melflux der Firma BASF, besonders bevorzugt Melflux 4930F, stabilisiert, indem eine bestimmt Konzentration wie zum Beispiel 0,4 mg Melflux pro mg Partikel in die Dispersion gegeben und für 15 Minuten gerührt wird. Anschließend wird die UV-Voraktivierung und Zellbehandlung wie in Beispiel 1 durchgeführt. Genau wie in Beispiel 1 beträgt die mittlere inhibitorische Konzentration (IC₅₀) der Partikel gegenüber den Tumorzellen 100-150 µg/ml, während die IC₅₀ der Partikel gegenüber nicht-malignen Zellen mindestens 200 µg/ml beträgt.

### Beispiel 3

Die Herstellung der Partikel wird wie im Beispiel 1 durchgeführt, jedoch werden zu den Edukten weitere Partikel, wie Silica- oder Eisenoxid-Silica-Schale-Partikel zugegeben. Dadurch werden Silica- oder Eisenoxid-Silica-Titandioxid-Kern-Schale Partikel erhalten. Die UV-Voraktivierung wird wie bei Beispiel 1 durchgeführt. Genau wie in Beispiel 1 beträgt die mittlere inhibitorische Konzentration (IC₅₀) der Partikel gegenüber den Tumorzellen 100-150 µg/ml, während die IC₅₀ der Partikel gegenüber nicht-malignen Zellen mindestens 200 µg/ml beträgt.

### Beispiel 4

Es werden kommerzielle TiO₂-Partikel (beispielsweise Degussa P25) mit einem Carbonsäurederivat, das mindestens eine Ethoxygruppe enthält, modifiziert. Vorzugsweise wird eine wässrige Mischung erzeugt, welche die TiO₂-Partikel in einer Konzentration von maximal 10 mg/ml, bevorzugt von 3 mg/ml, und das Carbonsäurederivat in einer Konzentration von 1 bis 100 mg/ml, bevorzugt von 6 bis 25 mg/ml enthält. Die Partikel werden mittels Ultraschall dispergiert. Die UV-Voraktivierung und Zellbehandlung wird wie in Beispiel 1 durchgeführt.

Genau wie in Beispiel 1 beträgt die mittlere inhibitorische Konzentration (IC₅₀) der Partikel gegenüber den Tumorzellen 100-150 µg/ml, während die IC₅₀ der Partikel gegenüber nicht-malignen Zellen mindestens 200 µg/ml beträgt.

## Patentansprüche

1. Photokatalytisch aktivierte Partikel mit einem durchschnittlichen Durchmesser zwischen 5 nm und 100 nm und einer Partikeloberfläche, die mit mindestens einem Carbonsäurederivat mit mindestens einer Ethoxygruppe modifiziert ist, wobei die Partikel mittels UV-Licht aktiviert wurden und durch die hierbei freigesetzten Radikale tumortoxische Eigenschaften aufweisen.

2. Partikel nach Anspruch 1, **dadurch gekennzeichnet, dass** die Partikel ein Halbleitermaterial, bevorzugt ein Halbleitermaterial ausgewählt aus der Gruppe bestehend aus
• Titanoxid, insbesondere Titanoxid in der Anatas- oder Rutil-Modifikation,
• Zinkoxid,
• Zinnoxid,
• Zirkonoxid,
• Cadmiumsulfid,
• Niobiumoxid
• und Mischungen hiervon
enthalten.

3. Partikel nach einem der vorhergehenden Ansprüche, **dadurch gekennzeichnet, dass** das mindestens eine Carbonsäurederivat maximal 15 Ethoxygruppen, bevorzugt maximal 4 Ethoxygruppen, enthält.

4. Partikel nach einem der vorhergehenden Ansprüche zur Verwendung als Arzneimittel, insbesondere zur Verwendung als Tumor-Therapeutikum oder zur Verwendung als antimikrobiell wirksame Substanz.

5. Partikel nach einem der vorhergehenden Ansprüche, **dadurch gekennzeichnet, dass** sie nach einer mindestens fünfstündigen Aktivierung gegenüber malignen humanen Zellen, bevorzugt Hypopharynxkarzinomzellen, besonders bevorzugt FaDu-Zellen, eine mittlere inhibitorische Konzentration IC₅₀ von maximal 150 µg/ml aufweisen.

6. Partikel nach einem der vorhergehenden Ansprüche, **dadurch gekennzeichnet, dass** die Aktivierung mittels UV-Licht ex-vivo erfolgt, bevorzugt erfolgt die Aktivierung dabei in einer wässrigen Dispersion.

7. Partikel nach Anspruch 6, **dadurch gekennzeichnet, dass**
die Aktivierung mit UVA-Licht, bevorzugt mit Licht des Wellenlängenbereiches von 315 bis 380 nm, besonders bevorzugt mit Licht der Wellenlänge 366 nm; und/oder
mit einer Intensität von 0,1 bis 2 mW/cm², bevorzugt 0,3 bis 0,5 mW/cm² und/oder
über einen Zeitraum von 5 bis 50 h, bevorzugt von 12 bis 24 h;
erfolgt.

8. Wässrige Dispersion von photokatalytisch aktiven Partikeln nach einem der Ansprüche 1 bis 7.

9. Verfahren zur Herstellung einer wässrigen Dispersion von photokatalytisch aktiven Partikeln, welches die folgenden Verfahrensschritte umfasst:
a) Herstellen eines Sols aus einer metallorganischen Verbindung, einer Säure und Wasser,
b) Umsetzen des Sols bei Temperaturen von 160 bis 200°C zu einem partikelhaltigen Gel,
c) Verdünnen des Gels zu einer Dispersion mit einer Partikelkonzentration zwischen 0 und 20 Gew.-% und
d) Bestrahlen der Dispersion mit UV-Licht.

10. Verfahren nach Anspruch 9, **dadurch gekennzeichnet, dass** die metall-organische Verbindung in Schritt a) ausgewählt ist aus der Gruppe bestehend aus
• Titanhalogeniden ausgewählt aus der Gruppe bestehend aus TiCl₄, TiOCl₂ und Mischungen hiervon
• Titanalkoxide, bevorzugt Titanalkoxide mit der allgemeinen Formel Ti(OR)₄, wobei R ein geradkettiger, verzweigter oder cyclischer Alkylrest mit 1 bis 6 Kohlenstoffatomen ist, besonders bevorzugt Titanalkoxide ausgewählt aus der Gruppe bestehend aus Titanium(IV)butoxid, Titanium(IV)ethoxid, Titanium(IV)propoxid, Titanium(IV)isopropoxid, Titanium(IV)tert-butoxid, Titanium(IV)methoxid sowie
• Mischungen hiervon.

11. Verfahren nach einem der Ansprüche 9 oder 10, **dadurch gekennzeichnet, dass** die Säure in Schritt a) ein Carbonsäurederivat ist, insbesondere ausgewählt aus der Gruppe bestehend aus Methoxypolyethylenglycol-Essigsäure, Methoxypolyethylenglycol-Propionsäure, O-Methyl-O'-succinylpolyethylenglycol, O-[2-(3-Succinyl-amino)ethyl]-O'-methyl-polyethylenglycol, O-(2-Carboxyethyl)-O'-methyl-undecaethylenglycol, 2-[2-(2-methoxyethoxy)ethoxy]-essigsäure, p-Toluolsulfonsäure und Mischungen hiervon, wobei die Säure in Schritt a) bevorzugt in einer Konzentration von 1 bis 100 mg/mL, besonders bevorzugt von 6 bis 25 mg/mL, vorliegt.

12. Verfahren nach einem der Ansprüche 9 bis 11, **dadurch gekennzeichnet, dass** dem Sol in Schritt a) weitere Partikel, bevorzugt Silica-Kern-Schale-Partikel oder Eisenoxid-Kern-Schale-Partikel, zugegeben werden.

13. Verfahren nach einem der Ansprüche 9 bis 12, **dadurch gekennzeichnet, dass**
das Gel in Schritt c) auf eine Partikelkonzentration von 0,1 bis 10 mg/mL, bevorzugt auf eine Konzentration von 1 bis 5 mg/mL, verdünnt wird; und/oder
der Dispersion in Schritt c) Polycarboxylether, bevorzugt in einer Menge von 0,4 mg/Partikel, zugegeben wird; und/oder
das UV-Licht in Schritt d) aus UVA-Licht, bevorzugt aus Licht des Wellenlängenbereiches von 315 bis 380 nm, besonders bevorzugt aus Licht der Wellenlänge 366 nm, besteht; und/oder
das UV-Licht in Schritt d) eine Intensität von 0,1 bis 2 mW/cm², bevorzugt von 0,3 bis 0,5 mW/cm², aufweist; und/oder.
die Dispersion in Schritt d) über einen Zeitraum von 5 bis 50 h, bevorzugt von 12 bis 24 h, bestrahlt wird.

14. Wässrige Dispersion von photokatalytisch aktiven Partikeln, herstellbar nach einem Verfahren gemäß einem der Ansprüche 9 bis 13.
